Europäisches Patentamt

European Patent Office

Office européen des brevets

(19)

(11) Publication number: **0 073 551**
**A2**

(12) **EUROPEAN PATENT APPLICATION**

(21) Application number: **82300023.7**

(22) Date of filing: **05.01.82**

(51) Int. Cl.³: **A 61 B 5/00**

(30) Priority: **27.08.81 US 296720**

(43) Date of publication of application: **09.03.83**
**Bulletin 83/10**

(84) Designated Contracting States: **BE DE FR GB IT NL SE**

(71) Applicant: **Becton, Dickinson and Company, Mack Centre Drive, Paramus New Jersey 07652 (US)**

(72) Inventor: **Burns, James Aloysius, 1104 Kipling Road, Elizabeth New Jersey (US)**

(74) Representative: **Ruffles, Graham Keith et al, MARKS & CLERK 57-60 Lincoln's Inn Fields, London WC2A 3LS (GB)**

(54) **Sample collection container.**

(57) An arrangement of serum separation tube 10 or other evacuated tube or container is provided which type of tube requires the addition of a variety of different additives, depending upon the use which is to be made of the tube and/or the test to be carried out once a sample of blood has been introduced. The invention contemplates a simplified form for adding reagents to different tubes by introducing by mass production techniques reagents as required simultaneously to separate pieces of paper and/or polyester. The separate pieces 18 with reagent thereon are subsequently introduced into individual separation containers or tubes as required. The introduced pieces will have the required quantity of reagent thereon for the particular test involved.

SAMPLE COLLECTION CONTAINER

BACKGROUND AND STATEMENT OF THE INVENTION

This invention relates generally to tubes and/or containers used to collect samples of blood for testing those samples for a variety of different conditions, which containers and/or tubes may be evacuated serum separation tubes, or microcollection containers utilizing a capillary flow to obtain a small sample of blood for subsequent testing. More particularly, this invention relates to such containers wherein reagents are introduced for imparting to the sample collected a particular condition for a specific test being carried out on that sample.

As will be appreciated by practitioners-in-the-art, a large number of procedures and/or applications have been developed in the past for introducing additives to serum separation tubes and/or other evacuated tubes or microcollection containers for receiving blood samples. These approaches include simply introducing the reagents in the form of liquids into the containers, coating the internal surfaces of the containers with certain reagents and so forth. Certain problems arise, however, with these approaches. First, with respect to the addition of liquids, it is difficult to control the separation procedures if liquids have been introduced into the containers prior to any separation procedure under centrifugation. Moreover, the application of reagents in liquid form introduces the problem of stability of the reagents over a period of time. It has been found that dried reagents have a much longer storage life, as will be appreciated.

The introduction of reagents by coating the internal surfaces of the containers introduce other problems in manufacturing in that the tubes must be handled in a production line on several passes for the introduction of various coatings. Moreover, one coating on the internal surface of a container may interfere with a subsequent coating so that the effectiveness thereof is reduced. As will be appreciated, such handling over a number of passes creates an expense problem in the manufacture of a plurality of such containers.

With this invention by contrast, an arrangement of a blood sample collection device is provided wherein reagents required for a variety of different such containers are introduced into the containers on separate pieces of paper, for example, or non-woven fiber such as polyester, etc. The size of the pieces of paper are such as to be easily insertable into the tubes or containers. The reagents, in the quantities required, are introduced onto the separate pieces of paper for subsequent introduction thereof into the tubes or containers, as required. As will be appreciated, by mass production of a plurality of such pieces of paper or non-woven thermoplastics, for example, large quantities of such reagents may be prepared for subsequent insertion as required into serum separation tubes or other evacuated tubes or containers or microcollection tubes. As will be appreciated further, more than one reagent may be introduced onto a separate piece for introduction, as required, if two or more reagents may be required for the subsequent test, once the blood sample is introduced into the container involved.

As purely illustrative of and representative of containers which may be utilized, in accordance herewith, separation tubes for separating and partitioning a fluid into separate phases may be used. As known in the art, the separation tube is provided with a barrier material having a specific gravity intermediate the specific gravity of the phases to be separated, and the material is caused to flow to the interface of the two phases and form a barrier therebetween upon the application of a force to separate the phases. Thus, for example, in separating whole blood into serum and cell phases, the barrier material is generally a mixture of a silicone fluid and hydrophobic silicon dioxide powder, with the mixture having a specific gravity intermediate the specific gravities of the serum and cell phases. Representative prior art in this connection is disclosed in U.S. Patent 3,852,194.

The present invention is directed to an improvement in the separation devices, for example, for separating a fluid into at least two phases by the use of a barrier material, wherein separate reagents are introduced, such as, for example, a blood anticoagulant such as sodium heparin or ethylenediamine tetraacetic acid. In such applications involving such serum separation tubes, the barrier material is positioned in the bottom closed end of the container. The barrier material does not interact with the fluid phases being separated, is substantially non-flowable at rest, and flows to the interface upon application of force, as discussed above. The material is generally a gel-like thixotropic material and is, preferably, comprised of a silicon fluid and a hydrophobic silica such as silicon dioxide. The material moves in the container in response to centrifugation to separate the fluid phases and stops flow at the interface between the phases as a result of its specific gravity. The barrier material generally has a specific gravity within the range of between about 1.035 and 1.6, and preferably within the range of between about 1.04 and 1.0555.

Once the barrier material has been introduced into the serum separation tube, a piece of paper or other material such as a polyester unwoven cloth is introduced into the container and may be positioned, for example, midway between the tube opening and the tube bottom. It may be fitted at an oblique angle and be circular in form to cooperate with the internal surface of the tube. The piece of introduced paper or non-woven thermoplastic material will have contained thereon a reagent for reacting, subsequently, with a blood sample introduced into the container.

The reagent may be, for example, diatomaceous earth as a blood coagulant. The quantity of diatomaceous earth introduced previously onto the piece which is subsequently introduced into the container will be according to the quantity of blood collected subsequently into the container. For example, 1-5mgs of diatomaceous earth may be introduced with a blood sample of 10ccs. Thus, the reagent introduced will serve as a powdered blood clotting accelerator subsequently for the testing procedure of the sample.

As further illustrative of the invention herein, one may note that the body of the materials introduced containing the reagents, in accordance herewith, may have a square configuration, may be in the form of a round disc, as discussed above, to conform to the internal surfaces of a tube such as an evacuated tube, or in the form of a strip. Moreover, the material may be a filter paper, for example, unwoven cloth such as a polyester, a tampon material, sintered polypropylene or other similar materials which will be useful in the context of an evacuated tube for the introduction of a blood sample. The material will, as will be appreciated, have a specific gravity which is greater than that of the blood corpuscles, and it will not be hemolytic, will not interfere with subsequent biochemical testing of the sample as required. Moreover, the material must be hydrophilic and possess sufficient rigidity and elasticity to maintain itself, if so required in a particular position while having sufficient pliability subsequently to be capable of moving under the action of centrifugal force during a separation procedure.

It will be appreciated, however, that this invention is not limited to the introduction of reagents on separate pieces of material into only serum separation tubes. For example, this invention contemplates, broadly, the introduction of such reagents into any blood collection type container wherein a sample of blood is to be subsequently tested according to certain procedures wherein an introduced reagent is required. For example, in microcollection tubes, wherein blood is collected from a surface puncture in the skin and the blood is collected through a capillary, a reagent may be introduced in accordance with this invention into a microcollection container therefore for a subsequent co-mingling with the sample collected for a subsequent test.

As purely illustrative of procedures which may be carried out in accordance with this invention, one may note the following table wherein a blood anticoagulant was introduced on several different types of filter paper of different sizes to react with a specific amount of blood. The reagent is the anticoagulant ethylenediamine tetraacetic acid (EDTA). It will be noted that a measurement was made of the quantity of EDTA actually absorbed onto the various different filter papers of different sizes. The ethylenediamine tetraamine was a 6% solution. In this procedure, different types of filter paper were cut into various sizes and shapes, and their sizes were re corded, in accordance with Table I below. Using a 20 microliter pipette, the EDTA was dropped onto the different size filters and the amount absorbed was recorded.

After this procedure, all filter papers were then placed in a desiccator for a 24-hour period. The filter papers were dropped into the labelled stoppered test tubes. It should be noted here, that some of the filter material was in the form of thimble-shaped filters while others were in the form of round pieces of material. All of the tubes, once the reagent containing substrates were introduced, were stoppered but not evacuated.

Subsequently, all of the tubes with the saturated filters were tested routinely in accordance with the SST (Serum Separation Test) test protocol TP-23-78-008 with the following procedures. Twenty milliliter syringes were used and the stoppers were removed from each tube and approximately 5ml of blood was dispensed into each tube. All the tubes were inverted four times and at 15-minute intervals to check for clotting. Inspection for clots or any form of coagulation was for a two-hour period.

TABLE I

| TYPE OF FILTER PAPER | SIZE OF FILTER PAPER | AMOUNT OF EDTA | AMOUNT OF BLOOD (APPROX.) | PREVENTED COAGULATION YES | NO |
|---|---|---|---|---|---|
| #603 Extraction | 32x5mm | .1ml | 5ml | | No |
| Thimble Single | 32x5mm | .22ml | 5ml | | No slight fibrin |
| Thickness | 32x8mm | .26ml | 5ml | Yes | |
| | 32x12mm | .3ml | 5ml | Yes | |
| Extraction Thimbles | 32x5mm | .4ml | 5ml | Yes | |
| 10x5mm | 32x8mm | .45ml | 5ml | | No |
| Double Thickness | 32x12mm | .5ml | 5ml | Yes | |
| Whatman Filter | 11x31mm | .8ml | 5ml | Yes | |
| Paper 100 Circles | 12x53mm | .9ml | 5ml | Yes | |
| 114 Wet Strengthened | 15x61mm | 1.6ml | 5ml | Yes | |
| Millpore Type HA 0.45 um | 45mm in Diameter | .26ml | 5ml | Yes | |

As will be seen, from the information contained in Table I, the diameter of the extraction thimble enabled it to move freely within the tube. However, the filter paper swelled and made the distance between the tube wall and the extraction thimble very close. It was established in the test carried out as represented by Table I that at least 0.26ml of EDTA is needed to prevent coagulation of a 5ml quantity of blood. Thus, it can be seen that standard filter paper can be quantitatively saturated with an anticoagulant and be used as an anticoagulator in a serum separation tube such as a VACUTAINER$^R$, a product of Becton Dickinson and Company.

As discussed above, while the invention contemplates filter paper as a preferred material for use as the substrate for containing a reagent for subsequent introduction into a test container, it is within the purview of this invention that any inert material such as cloth or polyester may be utilized as a substrate for containing the reagent, as long as it is sufficiently porous to absorb, or of a size to maintain the reagent introduced thereon. Moreover, the material must be such, as discussed above, that it will not interfere with any reactions which are to take place, subsequently, during a testing procedure, once blood samples are introduced into the container. Further, as discussed above, the size of the substrate for containing the reagent will vary depending upon the size and configuration of the container where the substrate is to be introduced. For example, for a microcollection tube, using a square substrate, the size may be, for example, 4.76mm x 4.76mm x 0.76mm in thickness. The quantity of reagent may be introduced to the extent where a resulting dried reagent quantity will be within the range of 0.36-
0.43mg dried. A broader range will be within the range
of 1.0-1.7mg dried. For a regular size VACUTAINER$^R$ for
example, the quantity of reagent may be within the
range of 5 and 30mg dried. In this connection, the substrate may be circular in form to conform to the inside
surfaces of the VACUTAINER$^R$ and may be, for example,
within the range of between about 9.525-12.70mm in diameter. Representative additives which may be utilized,
in accordance herewith, in addition to EDTA, include
sodium heparin, thrombin sodium fluoride, potassium oxalate, silica and sodium citrate. It will be appreciated,
that the reagent selected, will depend upon the kind of
test to be conducted subsequently upon the addition of
the blood sample to the container. For example, it has
been found that silica should be present on the substrate
in an amount to provide within the range of between about
3 and 7 parts for each 100 parts of a fluid barrier material for a serum separator tube.

Other objects and advantages of this invention
will be apparent from the following description, the accompanying drawings, and the appended claims.

DESCRIPTION OF THE DRAWINGS

Fig. 1 is a perspective view of a conventional
serum separator tube containing a disc with reagent
thereon, and illustrating the invention; and

Fig. 2 is a cross-sectional view of the serum
separation tube of Fig. 1 indicating the conditions
therein after a centrifuge application to the tube with
an introduced blood sample therein.

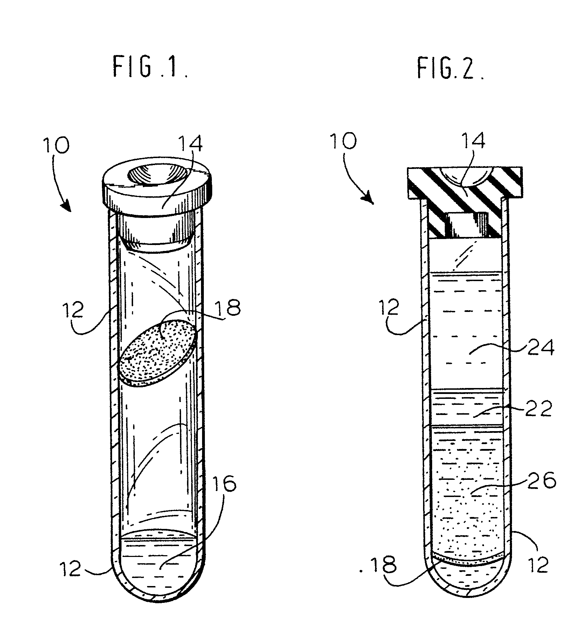

Referring to the drawings in which like reference
characters refer to like parts in the several views
thereof, in Fig. 1, a serum separation tube 10 is shown
with a stopper 14 closing the open end thereof. Adjacent the closed end, as can be seen in Fig. 1 is a thixotropic barrier material 22 which is introduced into the tube 12 for subsequent interaction with a blood sample for the separation of the blood sample into phases, as discussed above. Positioned in the end of tube 10 approximately midway therein between the open end and the closed end thereof is a disc 18 illustrating the invention herein. The disc will contain a desired reactant for a subsequent testing procedure for the blood sample. For example, the disc 18 may contain a quantity of diatomaceous earth for bringing about coagulation of the blood sample introduced. In this circumstance, the reagent introduced is a powdered reagent introduced onto the inert substrate which is polyester unwoven cloth. It may be positioned at an oblique angle, as shown in Fig. 1. The cap may be a butyl rubber cap which is inserted to sealingly engage with the open end of tube 12. The cap 14 may include a reduced central section 20 for easier insertion of a cannula for introduction of a blood sample, once the tube is evacuated. When the blood sample is introduced, the result is that the diatomaceous earth disperses within the blood and accelerates blood coagulation. Then the tube may be introduced to a centrifugation procedure for a period of time of perhaps ten minutes.

As can be seen in Fig. 2, once the application of centrifugal force has been applied to the tube, the blood sample has been separated into a blood serum 24, and a blood clot 26 with the thixotropic separating agent 22 positioned therebetween. Since, in this particular example the specific gravity of the reagent on the substrate 18, and the substrate polyester itself are both greater than the blood serum, they are not introduced into the blood serum layer which may be decanted easily in a high degree of purity without fibrin adulteration.

Thus, as can be seen from the above discussion, this invention provides a relatively easy procedure for introducing reagents into blood collection containers for subsequent reaction with a blood sample. Moreover, the arrangement, in accordance herewith, allows for a large variety of introduced reagents with easy selection for that introduction, depending upon the particular test for particular containers being prepared. It will be appreciated, by practitioners-in-the-art, that by using separate substrate pieces for receiving and maintaining the different reagents, different forms and combinations of reagents may be introduced onto the substrates for subsequent introduction into the tubes. Large quantities of substrate pieces may be prepared with reagents thereon in mass production procedures reducing the handling steps required in preparing blood sample containers such as serum separation tubes.

While the methods and forms of apparatus herein described constitute preferred embodiments of the invention, it is to be understood that the invention is not limited to these precise methods and forms of apparatus, and that changes may be made therein without departing from the scope of the invention which is defined in the appended claims.

CLAIMS

1.    A sample collection container, comprising a container defining a chamber and having an opening, together with a closure for the opening, characterized by a substrate material introduced through the opening, and a reagent on the substrate material for reacting with a sample introduced into the chamber, with the substrate material being inert to the reagent and to a sample introduced into the chamber, such that introduction of a sample into the chamber causes reaction between the reagent and the sample.

2.    A container according to claim 1 which is a blood sample collection container.

3.    A container according to claim 2, wherein the reagent is ethylenediamine tetraacetic acid, sodium heparin, potassium oxalate, silica, sodium citrate, thrombin or diatomaceous earth.

4.    A container according to claim 1 wherein the substrate material is porous for receiving a solution of the reagent, the reagent is introduced as a solution onto the substrate; and the solution is dried prior to the introduction of the substrate into the container.

5.    A container according to any preceding claim, wherein the substrate is filter paper, a non-woven thermoplastic, sintered polypropylene or a cloth material.

6.    A container according to any preceding claim, wherein the substrate is square, an elongate strip or circular in shape.

7. A blood serum separation tube, comprising an elongate container defining an evacuated chamber; a closed end and an open end for the chamber; a resilient stopper closing the opening; and a thixotropic gel material disposed adjacent the closed end, the gel having a specific gravity within the range of between about 1.035 and 1.60; characterized by a substrate material positioned in the chamber; a reagent on the substrate material for reacting with a blood sample introduced into the chamber; and the substrate material being inert to a blood sample introduced into the chamber, the gel and the reagent; such that introduction of a blood sample into the chamber causes reaction between the reagent and the blood sample.

8. A tube according to claim 7, wherein the reagent is ethylenediamine tetraacetic acid, sodium heparin, potassium oxalate, silica, sodium citrate, thrombin or diatomaceous earth.

9. The tube according to claim 7 or 8, wherein the substrate material is porous for receiving a solution of the reagent, the reagent is introduced as a solution onto the substrate; and the liquid reagent is dried prior to the introduction of the substrate into the container.

10. A tube according to any of claims 7 to 9, wherein the substrate is filter paper, a non-woven thermoplastic, or a cloth material.

11. A tube according to any of claims 7 to 10, wherein the substrate is circular in form and of a size to cooperate engagingly with the internal walls of the chamber.

12. A tube according to claim 11, wherein the circualar substrate is of a size to remain fixed in a position about midway between the closed and open ends prior to introduction of a blood sample.

13. A tube according to claim 12, wherein the reagent is diatomaceous earth.

14. A container according to claim 2, wherein the blood sample collection container is a microcollection container; the substrate is a porous filter paper; and the reagent is a blood anticoagulant.

15. A container according to claim 14, wherein the blood anticoagulant is ethylenediamine tetraacetic acid.

16. A container according to claim 15, wherein the ethylenediamine tetraacetic acid is dried and is present in an amount within the range of between 1.0 and 1.7 mgs.

17. A tube according to claim 12, wherein the reagent is silica present in an amount within the range of between 3 and 7 parts for each 100 parts of the gel material.

FIG.1.

FIG.2.